# EUROPEAN PATENT SPECIFICATION

(11) **EP 3 717 037 B1**
(45) Date of publication and mention of the grant of the patent: **15.03.2023**
(21) Application number: 18888066.0
(22) Date of filing: 11.12.2018
(51) Int. Cl.: A61M 1/14, A61M 1/36, A61M 37/00, A61M 1/00, A61M 1/34

(54) **AN ASPIRATION DEVICE**
ABSAUGVORRICHTUNG
DISPOSITIF D'ASPIRATION

(30) Priority: 13.12.2017 TR 201720265
(43) Date of publication of application: 07.10.2020
(73) Proprietor: Dokuz Eylul Üniversitesi Rektorlugu, 35210 Izmir (TR)
(72) Inventor: SILISTRELI , Erdem Erinç, Güzelbahçe/ Izmir (TR); ER, Ahmet Salih, i li/ zmir (TR)
(86) International application number: PCT/TR2018/050785
(87) International publication number: WO 2019/117840

(56) References cited:
- US-A1- 2002 110 485
- US-A1- 2004 195 178
- US-A1- 2009 032 469
- RAM HARISH ET AL: "The AngioVac Device and Its Anesthetic Implications", JOURNAL OF CARDIOTHORACIC AND VASCULAR ANESTHESIA, vol. 31, no. 3, 1 June 2017 (2017-06-01), pages 1091-1102, XP085122601, ISSN: 1053-0770, DOI: 10.1053/J.JVCA.2016.12.017
- GLASG ET AL: "In Vitro Model for the Evaluation of Inferior Vena Cava Filters: Effect of Experimental Parameters on Thrombus-Capturing Efficacy of the Vena Tech-LGM Filter", JOURNAL OF VASCULAR AND INTERVENTIONAL RADIOLOGY, ELSEVIER, AMSTERDAM, NL, vol. 9, no. 2, 1 March 1998 (1998-03-01), pages 295-304, XP023182126, ISSN: 1051-0443, DOI: 10.1016/S1051-0443(98)70272-6 [retrieved on 1998-03-01]

## Description

### Technical Field of the Invention

The invention is related to an aspiration device that has been developed in order to be used in acute pulmonary thrombo-emboli. and thrombosis treatment.

### Known State of the Art (Prior Art)

There are two hazardious consequences of deep venous thrombosis:
One of these dangers is when the thrombosis gets detached and the artery of the lung is blocked (Acute Pulmonary Thrombo-emboli), and the other danger is when the thrombosis gets larger towards the top and leads to Massive Iliocaval Thrombosis which clogs all of the central venous system. Sometimes medical treatment is not enough to treat these two diseases. Surgical process necessitates long term preparation and is risky.

The American aspiration device (AngioVac) used in the known state of the art is a gathered system; and the lines are long and they pass outside the surgical area, and the pump and filter is located externally. As the filter gets clogged quickly, reverse circulation is obligatory and due to the length of the lines, it is mandatory for higher volumes to be loaded to the patient. 275 cm3 volume is present per 1 meter of the lines through which blood passes and this one unit is a little lower than the volume of the blood prepared for transfusion. If the line is a little longer than 2 meters, this means that 2 units of blood needs to be prepared and used before the process, and the length necessary in the system (although it may change according to the equipment) is above 3-4 meters. It may be necessary to reverse the flow due to clogging of the lines from time to time and to air leakage and to refill the lines with blood. It is therefore an important problem for the lines to be long and the blood volume loss is higher if the lines are longer. In such a case when the flow is reversed, the amount of blood and blood products used shall increase incrementally.

It is mandatory to have a centrifugal pump head in AngioVac and this pump head is not available in every surgical operation room. As a solution, a ready to use centrifugal pump head is kept ready. However the sales price of the centrifugal pump that is necessary for AngioVac usage is quite high. On the other hand, it is very costly to carry and deliver the same pump to different locations in Turkey. Additionally some cases need to be postponed or cancelled.

The usage of AngioVac is difficult and quite expensive, and it is a system which is not compatible technically with the operation rooms in Turkey.

An aspiration device having shorter lines for blood circulation and having less space consuming pumps and lines has been developed by means of the invention. Ram et al, 10.1053/ J.JVCA.2016.12.017 discloses an aspiration device for the treatment of pulmonary embolism by drawing out thrombi, made of 2 cannulas at each extremity of the device. The centrifugal pump head pulses blood withdrawn from the body; the blood being filtered and returned to the patient with a 16F cannula. A controlled suction of blood is performed.

### Brief Description of the Invention and its Aims

The present invention is limited to the appended set of claims.

The invention is related to an aspiration device formed of a compact and original set which can remove large thrombi from the circulation, developed in order to be used in treating two dangerous diseases called Thrombo-emboli and Massive I liocaval Thrombosis.

By means of the device, massive thrombi are removed with a flexible canulla inserted into the central vein in the iliofemoral vein system, the pulmonary artery (arteries of the lungs) and vena cava (central vein system), wherein said thrombi are filtered through a suitable pored filter and are returned back to circulation from another central vein by means of a centrifugal pump.

Said device is compact which can be placed in the operation room as a whole and all of its lines, filter and motor unit can also be fitted in the operation room. As a result all of the equipment and perfusionist adjustments can be carried out in a controlled manner.

In this device the lines through which blood circulates are shorter and as it is more compatible with physiology and is more controlled, less volume is loaded to the patient and hemodynamic controls can be carried out easily. In this device, the problems of clogging of the lines the obligation of reverse circulation is eliminated and the amount of blood and blood products used is reduced. Every 1 meter of shortening of the lines means less usage of an additional volume consisting of a 275 cm³ blood and blood products and in this device the compact line usage enables a few less units of blood to be used per case. Moreover if the lines are clogged the flow needs to be reversed from time to time and the lines may need to be emptied and refilled. As a result, blood loss in short lines are significantly reduced.

This device comprises a sterile set which can be used once and disposed of. The pump which provides blood circulation in this device is centrifugal and the electric motor that can provide motion to the pump head can be used in several cases. Said motor is a deployable equipment and can be positioned within the operating table. The shortness and compactness of the lines and property of easyly manipulation by the surgeons can only be achieved with a motor that designed within a system that can be kept within the operating table. This allows avoiding problems such as expensive motor usage, travel organization, stacking and high costs.

Said device has practical usage and it is more compatible with systems used in the operation rooms in Turkey.

The device developed by means of the invention is less expensive in comparison to the device mentioned in the known state of the art.

### Definition of the Figures of the Invention

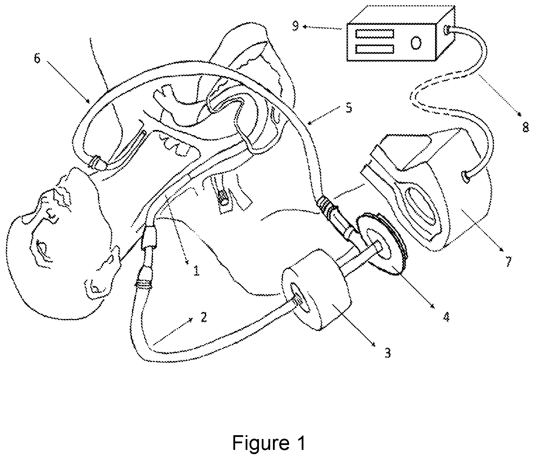

- **Fig. 1.**: A thrombosis aspiration, filtering and centrifugal circulation system which can be set up within the operating table.

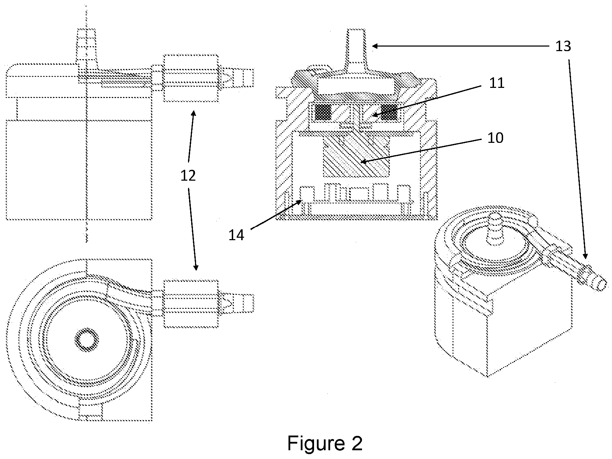

- **Fig. 2.**: Pump motor, housing and contents that can be locked to the centrifugal pump head and can be kept inside the surgical operating table inside a sterile cover.

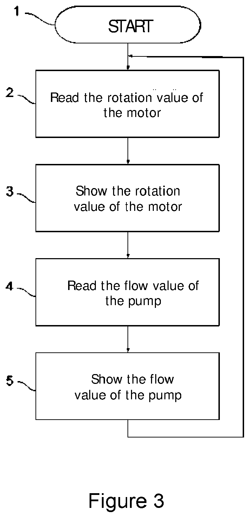

- **Fig. 3.**: Flow diagram of the software embedded to the hardware in the microcontroller unit located in the control unit.

### Definitions of the parts/ aspects/ sections forming the invention

- **1.**: Any kind of cannulla which can withdraw thrombosis.
- **2.**: Pipe line (inflow) through which thrombotic blood is received.
- **3.**: Thrombosis filter
- **4.**: Centrifugal pump head
- **5.**: Reverse flow (outflow) of the filtered blood and blood cleaned from thrombosis back to the body
- **6.**: A 16 F cannulla through which filtered blood is transferred to the body
- **7.**: A pump motor that can be taken into the operating table which can be locked onto the centrifugal pump head, found inside a compact mould with all its unit including the flow meter
- **8.**: Extension cord comprising power, data and control lines
- **9.**: A microcontroller unit having special software and control unit: The rate per minute data output of the motor and the data output (litre/minute) data of flow meter transducer are sent via a cable to the control unit placed outside the operating table. A software which shows the centrifugal pump head flow (litre/minute) values and the rotational value (RPM) of the motor in the system being embedded to the hardware of the microcontroller unit, that is present in the control unit.
- **10.**: Brushless DC type electric motor and encoder unit
- **11.**: Magnetic Coupling unit and magnetic disc which transmits the rotational motion to the centrifugal pump head by means of the magnetic rotational system.
- **12.**: A flow meter transducer unit consisting of ultrasonic resonators using the time difference method.
- **13.**: Centrifugal pump head
- **14.**: Motor drive circuit

### Detailed Description of the Invention

The invention is an aspiration device having a short and compact line system that has been developed in order to be used in thrombosis aspiration from large veins and the pulmonary artery.The purpose of the shortness of lines is to eliminate the problems such that clogging of the lines and the obligation of reverse circulation and thus to reduce the amounts of blood and blood products usage.

The aspiration device subject to the invention that has been developed in order to be used in acute pulmonary thrombo-emboli and massive iliocaval thrombosis treatment comprises, any kind of cannulla that is used in drawing out thrombi, a short and compact pipe line through which the thrombotic blood is received, thrombus filter, centrifugal pump head, return line for the filtered and blood cleaned from thrombosis to be returned to the body, cannula which supplies the filtered blood back to the body (preferably 16F cannulla), a pump motor which is portable and can be locked to the centrifugal pump head having all units thereof together with the flow meter located inside a compact mould, extension cord comprising the power, data and control lines and a microcontroller with special software.

In the device subject to the invention a brushless DC type electric motor is used. The centrifugal pump head is a part that is available in the set that is received into the operating table and the rotation motion to this head is provided to this head by means of the electric motor using the magnetic coupling system. Pump motor, housing and contents that can be locked to the centrifugal pump head can be deployed inside the surgical operating table inside a sterile cover. A flow meter transducer unit consisting of ultrasonic resonators using the time difference method can also be found inside this mould having the motor unit located therein. The rate per minute (rpm) data output of the motor and the data output (litre/minute) data of flow meter transducer are sent via a cable to the control unit placed outside the operation room. The hardware of the microcontroller unit, that is present in the control unit shows the centrifugal pump head flow (litre/minute) values and the rotational value (RPM) of the motor in the system.

The sets which comprise the filter and the centrifugal head, through which the blood flows are disposable and the motor and control units are fixtures that cannot be sterilized. As the motor is portable the same motor can be used in several cases.

## Claims

1. An aspiration device that has been developed in order to be used in acute pulmonary thrombo-emboli and massive iliocaval thrombosis treatment **characterized in that** it comprises, any kind of cannulla that is used in drawing out thrombi, a pipe line through which the thrombotic blood is received, thrombus filter, centrifugal pump head, return line for the filtered and blood cleaned from thrombosis to be returned to the body, cannula which supplies the filtered blood back to the body (preferably 16F cannulla), a pump motor which is portable and can be locked to the centrifugal pump head having all units thereof together with the flow meter located inside a mould, extension cord comprising the power, data and control lines and a microcontroller with software.

2. An aspiration device according to claim 1, **characterized in that** the sets comprising the filter and the centrifugal head are disposable.

3. An aspiration device according to claim 1, **characterized in that** it comprises a 16 F cannulla through which the filtered blood is transferred to the body.

## Patentansprüche

1. Absaugvorrichtung, die entwickelt wurde, um bei der Behandlung von akuten pulmonalen Thromboembolien und massiven iliocavalen Thrombosen verwendet zu werden, **dadurch gekennzeichnet, dass** sie jede Art von Kanüle, die beim Herausziehen von Thromben verwendet wird, eine Rohrleitung, durch die das thrombotische Blut aufgenommen wird, Thrombus-Filter, einen Zentrifugalpumpenkopf, eine Rücklaufleitung für das gefilterte und von Thrombosen gereinigte Blut, das in den Körper zurückgeführt wird, Kanüle, die das gefilterte Blut in den Körper zurückführt (vorzugsweise 16-F-Kanüle), einen Pumpenmotor, der tragbar ist und am Zentrifugalpumpenkopf befestigt werden kann, wobei deren alle Einheiten zusammen mit dem Durchflussmesser in einer Form untergebracht sind, ein Verlängerungskabel, das die Strom-, Daten- und Steuerleitungen, und einen Mikrocontroller mit Software enthält.

2. Eine Absaugvorrichtung nach Anspruch 1, **dadurch gekennzeichnet, dass** die den Filter und den Zentrifugalkopf umfassenden Sätze Einwegartikel sind.

3. Eine Absaugvorrichtung nach Anspruch 1, **dadurch gekennzeichnet, dass** sie eine 16-F-Kanüle umfasst, durch die das gefilterte Blut in den Körper geleitet wird.

## Revendications

1. Dispositif d'aspiration qui a été développé pour être utilisé dans le traitement des thrombo-embolies pulmonaires aiguës et des thromboses iliocaves massives, **caractérisé en ce qu'**il comprend, tout type de canule qui est utilisée pour aspirer les thrombus, une conduite par laquelle le sang thrombotique est reçu, un filtre à thrombus, une tête de pompe centrifuge, une ligne de retour pour le sang filtré et nettoyé des thromboses à renvoyer au corps, une canule qui renvoie le sang filtré vers le corps (de préférence une canule 16F), un moteur de pompe qui est portable et peut être verrouillé à la tête de pompe centrifuge dont toutes les unités, ainsi que le débitmètre, se trouvent à l'intérieur d'un moule, une rallonge comprenant les lignes d'alimentation, de données et de commande et un microcontrôleur avec un logiciel.

2. Dispositif d'aspiration selon la revendication 1, **caractérisé en ce que** les ensembles comprenant le filtre et la tête centrifuge sont jetables.

3. Dispositif d'aspiration selon la revendication 1, **caractérisé en ce qu'**il comprend une canule 16 F à travers laquelle le sang filtré est transféré vers le corps.
